# EUROPEAN PATENT SPECIFICATION

(11) **EP 2 939 661 B1**
(45) Date of publication and mention of the grant of the patent: **23.11.2022**
(21) Application number: 13869051.6
(22) Date of filing: 27.12.2013
(51) Int. Cl.: A61K 9/16, A61K 47/26, A61K 31/138, A61K 31/445, A61K 31/519, A61K 31/4045, A61K 31/4178

(54) **NOVEL MICROGRANULAR FORMULATION**
NEUARTIGE MIKROGRANULÄRE FORMULIERUNG
NOUVELLE FORMULATION DE MICROGRANULES

(30) Priority: 31.12.2012 KR 20120158709
(43) Date of publication of application: 04.11.2015
(73) Proprietor: CorePharm Co., Ltd., Sujeong-gu, Seongnam-si, Gyeonggi-do (KR); CorePharmbio Co., Ltd., Sujeong-gu, Seongnam-si, Gyeonggi-do, (KR)
(72) Inventor: GOO, Young Sam, Cheongju-si Chungcheongbuk-do 361-805 (KR); KIM, Jeong Tae, Seoul (KR)
(74) Representative: Louis Pöhlau Lohrentz
(86) International application number: PCT/KR2013/012346
(87) International publication number: WO 2014/104840

(56) References cited:
- EP-A2- 2 368 546
- WO-A1-93/09767
- WO-A1-93/25204
- WO-A1-2005/097070
- WO-A2-2004/108089
- WO-A2-2004/108089
- WO-A2-2011/077451
- US-A1- 2003 175 355
- US-A1- 2009 155 360
- US-A1- 2010 034 891
- US-A1- 2011 244 050
- US-A1- 2011 311 623
- US-A1- 2012 052 098

## Description

### Technical Field

The present invention relates to a microgranule preparation which can be taken without water, and rapidly dissolves when administered orally. More specifically, it relates to the preparation that minimizes the amount of residual remained in a packing vessel or wrapping paper upon administering, and can exactly regulate the amount of a drug distributed in the wrapping paper of an unit dosage in a preparation process, the preparation is the microgranule preparation prepared by coating the drug on the inert-core and post-mixing it with sugar or sugar alcohol. The microgranule preparation of the present invention is characterized in that it does not have any feeling of irritation and aftersensation in a mouth, when administered orally, and furthermore it can be rapidly disintegrated and dissolved in the mouth, together with a reduction of the amount of residual in the wrapping paper and the exact regulation for a contents of the preparation distributed in the wrapping paper.

### Background Art

A tablet or capsule is the most preferred form of administration in the pharmaceutical preparations to be administered orally, but if a patient has a difficulty in swallowing or needs to take it without water, the preparation which rapidly disintegrates and/or dissolves in a mouth can be considered.

In this case, in general, a tablet which rapidly disintegrates in a mouth (orodispersible tablet, ODT (orally disintegrating tablet)) or film preparations which rapidly dissolves in a mouth (strip preparation in the mouth, ODF (oral disintegrating film)) can be considered. However, in the case of ODT, it commonly takes at least 30 seconds to disintegrate and dissolve completely in a mouth. In particular, since a severe feeling of irritation is felt in a mouth until the disintegration is completed, although it can be taken without water, it does not greatly increase the patient's convenience for taking a drug.

In the case of ODF, it is the preparation which is produced by loading an effective ingredient to a strip with a film type, and used as a mouthwash, etc., but basically there is a limitation on the amount of effective ingredient loaded in the preparation, and also because the macromolecule which forms the film is cohesive, there is a displeasure due to the feeling of irritation and aftersensation in a mouth and so the patient's inconvenience is caused. Meanwhile, in relation to the granular preparation which is administered orally, there is an example that a herbal medicine extract is made into the granule agent, but it is not the preparation which rapidly dissolves in a mouth after the administration, it must be administered with water, and also, the feeling of irritation in a mouth is very great, so it has been considered as a preparation apart from one enhancing the patients' convenience for taking the drug. Also, it has been considered that the existing granular preparations are suitable for mixing with a drink or taking with food and has been less considered as an independent alternative means for the oral administration which can be replaced with a tablet or capsule. In addition, in the technical field to which the present application subjects, it is common that the granule is considered as an intermediate necessary for tableting the tablet or filling the capsule, and it is common knowledge that the convenience for taking the granule is greatly decreased when making it as the final administration preparation. Accordingly, it is difficult to find an example for increasing the convenience of taking the drug by making the granule as an independent administration preparation.

In particular, it can be made an attempt on the convenience for taking the drug by making the granule or excipient for effective ingredients to a size smaller than the conventional granule, for example, a powder, but in this case, it faces a problem caused from a preparation such as the powder, the problem is that it is hard to administer the exact amount of the powder when the patient takes it.

In other words, in the case of a tablet or capsule which is an ordinary oral administration preparation, a unit dose preparation is easily separated from a packing vessel, but in case of a powder, there would be a great mass left in the packing vessel. Therefore, in case of prescription only medicine which needs a professional treatment and the therapeutic effect can be greatly differed according to the administrative dose, not the case of general pharmaceuticals such as a vitamin, etc., when it is made as a powder, there are problems that a patient does not take all of medicines included in the wrapping paper, and some of the medicines are remained in the wrapping paper. In addition, in the manufacturing process, since there is a limitation on a fluidity of the preparation, there were many cases in which the amount of the medicine to be inserted differs in each wrapping paper, and thus, there was a problem that the contents of the effective ingredients are substantially varied in each unit dose form.

US 2011/0244050 A1 is directed to a pulsed-release sildenafil composition and method for preparing said composition.

### Disclosure

### Technical Problem

Therefore, the object of the present invention is to solve the problems mentioned above, i.e., to provide means for minimizing the amount of the preparation remained in the wrapping paper, and also, for making better the fluidity of the preparation for each unit dose form to have the exact contents of the effective ingredients. Also, furthermore, the problem for solving in the present invention is to provide new preparations in which the dissolution rate in a mouth is fast, and the feeling of irritation and aftersensation in the mouth are not present.

### Technical Solution

To solve the above-mentioned problems, according to the present invention, there is provided a technical means as follow:
A composition of microgranule preparation for oral administration according to claim 1 is provided.

In the above preparation, the microgranule preparation is provided, the preparation is characterized in that the inert core is preferably sugar or sugar alcohol.

In the above preparation, the microgranule preparation is provided, the preparation is characterized in that sugar or sugar alcohol is preferably selected from the group consisting of xylitol, mannitol, isomalt, sorbitol, maltitol, the fined white sugar, lactose, inositol, erythritol, crystaline fructose, trehalose, ribitol, arabitol, galactitol, lactitol and maltotritol.

### Advantageous Effects

According to the present invention, there can be provided with a microgranule preparation for oral administration which rapidly dissolves in a mouth when orally administered, the preparation has effects in which the effect for masking bitterness of a drug with a bitter taste is excellent, and there are no feeling of irritation and aftersensation in the mouth after the administration, unlike the existing general powder or granule.

### Description of Drawings

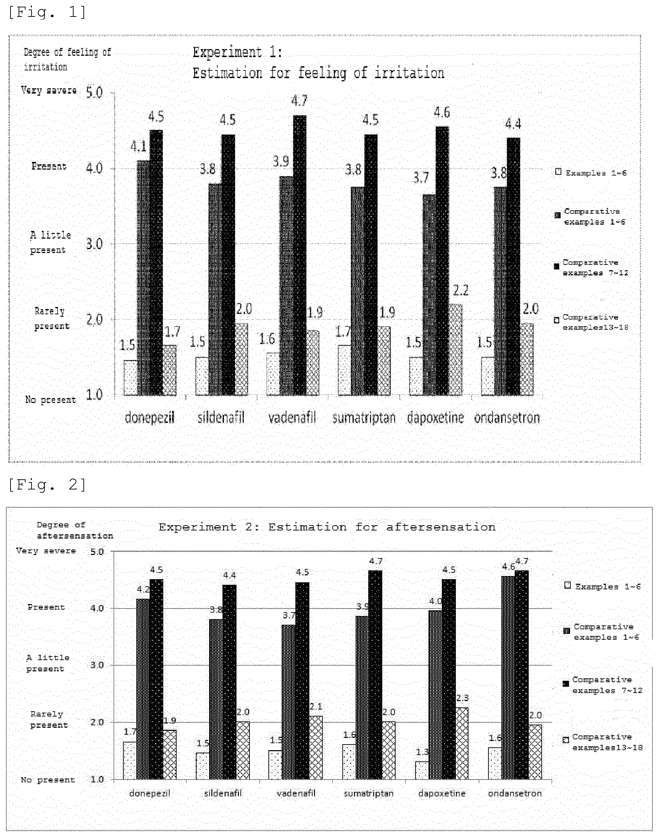

Fig. 1 shows an evaluation result of feeling of irritation.
Fig. 2 shows an evaluation result of aftersensation.

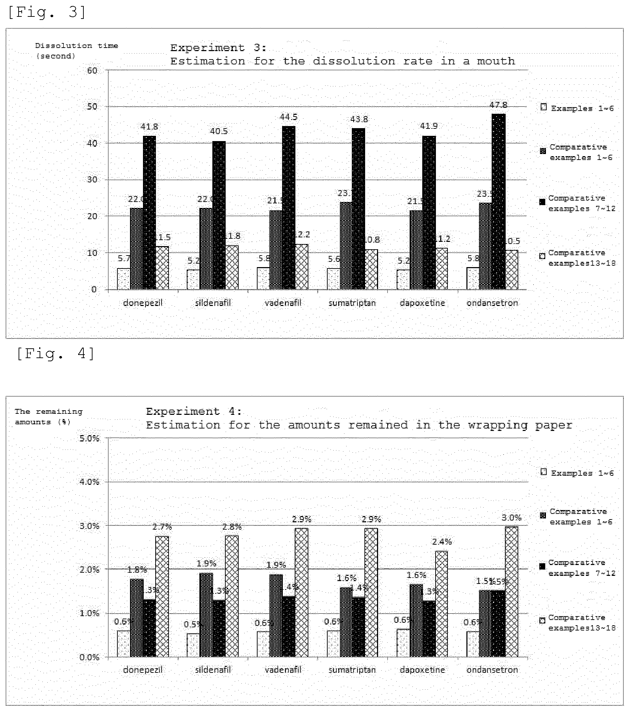

Fig. 3 shows an evaluation result of dissolution rate in a mouth.
Fig. 4 shows an evaluation result for amounts of the residual in a wrapping vessel.

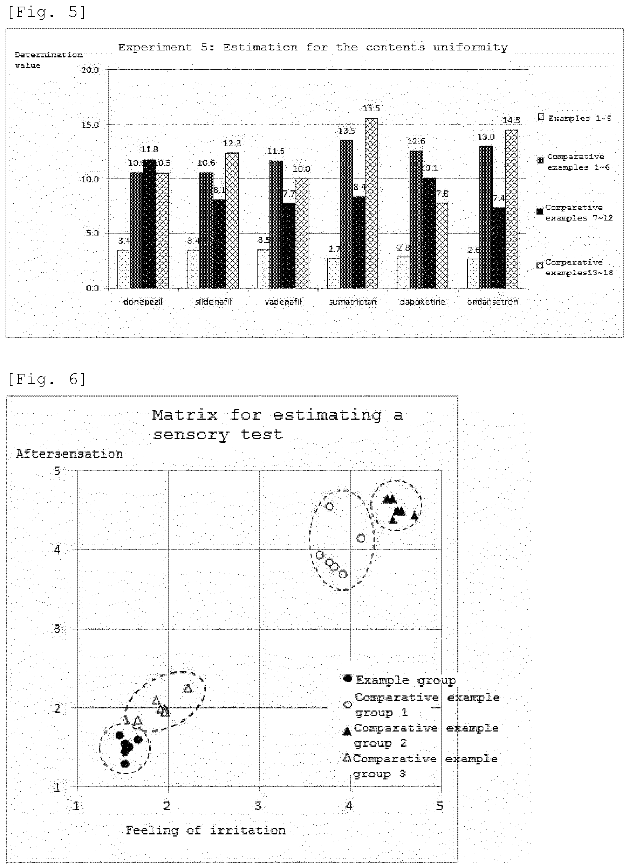

Fig. 5 shows an evaluation result for uniformity of the contents.

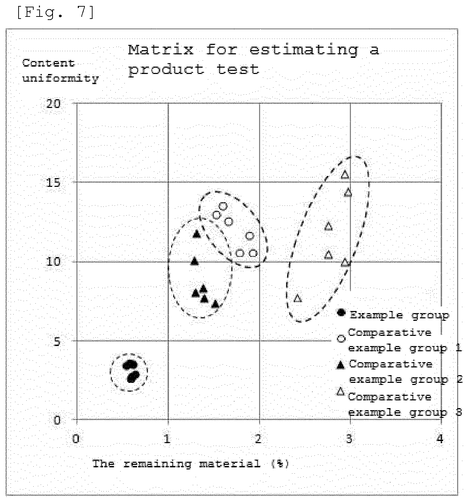

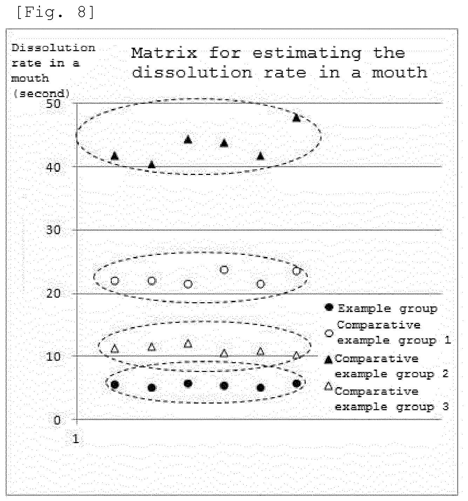

Figs. 6 to 8 show an overall examination evaluation for examples and comparative example.

### Best Mode for Invention

The term, 'microgranule preparation' is used as the meanings commonly referring to, but not limiting to, a powder, a micro-granule and a granule defined by the Korean Pharmacopoeia, and means a granular preparation made of the small fine or general particles.

In particular, the microgranule preparation of the present invention is constituted by mixing an inert core coated with a drug layer including the effective ingredient and sugar or sugar alcohol.

The term, 'drug' as used in the present invention means an effective ingredient which can be included in the preparation of the present invention and is pharmacologically active. The present invention is one characterized in the preparation itself, and has a basic premise that an effective ingredient can be included in the preparation of the present invention, and thus, the effective ingredient is not limited.

As the effective ingredient which is included in the present invention Sildenafil, Tadalafil, Udenafil, Donepezil, Glymepide, Dexibuprofen, Pranlukast , Desmopressin, Acetaminophen, Pitavastatin , Rebamipide, Azithromycin, Pseudoephedrine HCl, Ranitidine HCl , Levocetirizine HCl is listed and the pharmaceutical acceptable salt thereof can be also included.

The term, 'sugar or sugar alcohol' as used in the present invention means the material which can be dissolved rapidly when orally administered and is used in a raw material of the inert bead and in post-mixing. As the specific example, the pharmaceutically acceptable sugar or sugar alcohol such as xylitol, sucrose, mannitol, isomalt, sorbitol, maltitol, the refined white sugar, lactose, inositol, erythritol, crystaline fructose, trehalose, ribitol, arabitol, galatitol, lactitol and maltotritol, etc. and the mixtures thereof can be mentioned, but not limited to them.

In the present invention 'inactive core' means a material that is not pharmaceutically active and can coat a drug layer. A globular bead having a size of approximately 50 ~ 400 um can be used. As the specific example, sugar or sugar alcohol mentioned above such as a sucrose bead can be used, but is not limited to it.

The feeling of irritation as used in the present specification refers to the feeling to which a patient who has taken a medicine causes unpleasure due to that the patient notices the medicine as a foreign substance after administering it, and comprises, for example, a prickly feeling such as a sand, or a feeling irritating a mucosa of a mouth or tongue, and also a sticky feeling such as a mucus substance.

An aftersensation as used in the present specification means that although a substantial amount of time has been passed after administering the preparation, for example, despite that about 20 seconds have been passed after orally administering, and then the preparation comprising the effective ingredients has already dissolved and absorbed into the body, the sensation that a preparation or a part of it is remained in the mouth or the sensation related to a trace of taking the medicine such as a taste or feeling for the preparation is not yet removed in the mouth is maintained within the mouth, and means the sensation causing a displeasure depending on the patient, although it does not correspond to the feeling of irritation. When such aftersensation is present, it can result in a desire to additionally drink water or a drink, and in this case, since the feature of the preparation characterized in taking it without water is not exerted, it must be considered to improve the quality of the preparation together with the feeling of irritation.

Hereinafter, the present invention will be specifically explained.

When the effective ingredients are prepared in the form of powder or granule and then are administered, various limitations such as a dosage, a presence of aftersensation or feeling of irritation within a mouth, masking of bitterness, etc., are present, but, the problem that the preparation is remained in the wrapping paper cannot be overlooked.

That is, in the case of a tablet or capsule, it is easily separated from the wrapping paper, and when being separated, the content loss rarely occurs. But in the case of powder or granule, since the substantial amount of the medicine is remained in the wrapping paper, the administering dose of the effective ingredient is eventually varied and thus, the possibility affecting the treatment effect cannot be ignored due to the variation. In particular, in order to prevent this problem, it is known to the prior art the method that assembles an effective ingredient and excipient and then make them to the granule, not to simply mix them. However, although they are made into the granule, since the granule can be pulverized by a mild impact when being stored, the basic problem that the effective ingredient is remained in the wrapping paper at the time of administration due to the occurrence of fine particles during the storage of it could not be resolved.

The present invention is made based on the grounds of this problem, and in a medicine preparation in which the powder or granule is inserted into the wrapping paper and then supplied, as a result of carefully studying ways for all of the medicine to be discharged without being remained in the wrapping paper when the patient takes it, it was found that when the drug layer is coated on the inert core, unlike the existing powder or granule which is made by simply mixing the effective ingredient and excipient or assembling the effective ingredient to obtain the granule, since fine particles are rarely occurred, an impact resistance is excellent and the fluidity is better, there is little amount of the medicine remained in the wrapping paper when the patient receives it.

In particular, since the novel microgranule preparation of the present invention has the excellent impact resistance and also has a great fluidity, the exact amount can be distributed to each packing unit in the manufacturing process, and thus, it has very advantageous effect in constituting the medicine of which administering dose is relatively small as a form of powder.

The microgranule preparation of the present invention is made by coating the drug layer comprising the effective ingredient on an inert core, for example, sucrose bead, and then mixing it with sugar or sugar alcohol.

More specifically, the effective ingredient is dissolved or dispersed in the coating solution consisting of the coating base and the solvent. The result is yielded by flowing the inert core consisting of sugar or sugar alcohol, that is, the global bead in a flow-bed coating machine and simultaneously coating it with the coating solution comprising the effective ingredient made previously by a bottom spray manner. Additionally, a final mixture is obtained by mixing the result with sugar or sugar alcohol.

The microgranule preparation obtained by said method has an effect that the amount remained in the wrapping paper is minimized and also the disintegration rate in the mouth is rapid, and the aftersensation and feeling of irritation in the mouth are not present.

The feeling of irritation in a mouth refers to the feeling that causes the unpleasure to the human taking the medicine due to that the human appreciates it as a foreign material in the mouth, when administered it, according to the present inventors' research, such feeling of irritation was founded as being closely related to the microscopic disintegration and dissolution pattern of the preparation administered in the mouth. That is, in order to reduce feeling of irritation, it was found that the integration and dissolution in the mouth should be started simultaneously with administering the preparation, and also the disintegration and dissolution rates in the mouth should be very constant.

To accomplish this, the present invention uses sugar or sugar alcohol as an inert core, and by taking such constitution, when the preparation is administered the integration and dissolution are started instantaneously in the mouth, the rapid and continuous disintegration and dissolution are occurred within the microscopic period from several seconds to dozens of seconds, and eventually the new preparation without any feeling of irritation is obtained.

In addition, according the research of the inventors of the present application, it was noticed that the use of an inert core affects the aftersensation in the mouth, and identified that there is not any feeling in the mouth due to the administration of the preparation by moving instantaneously to a gastrointestinal tract together with saliva without remaining in the mouth the inert core which is included in the preparation together with the effective ingredient and sugar or sugar alcohol to be post-mixed.

In particular, the microgranule preparation of the present invention has characteristic features that can be disintegrated and dissolved rapidly in a mouth, and such effect is also considered as being an effect obtained by using sugar or sugar alcohol as an inert core. That is, in the case of other preparation taking without water like the present invention, for example, ODF or ODT which can be commercially used, it remains in a mouth at least dozens of seconds after the administration, but the preparation of the present invention is completely disintegrated and dissolved within several seconds (substantially at the same time as the administration) and then moved to the gastrointestinal tract, and this is differentiated from the existing 'preparation being administered without water'.

### Embodiment for Practicing the Invention

Hereinafter, the present invention will be explained via working examples. However, it should be noted that the following examples do not limit the scope of the rights of the present invention.

### Example 1 to 6

According to Table 1 as below, various effective ingredients are dissolved or dispersed in the coating solution consisting of the coating base and solution. The result is obtained by coating a globular bead (diameter of particle: 50 ~ 400 µm) consisting of sugar or sugar alcohol with the previously prepared coating solution comprising an effective ingredient in a bottom spray manner, with flowing the globular bead in a fluid coating machine (the temperature of wind: 50°C). Additionally, the final mixture is obtained by mixing this result with sugar or sugar alcohol.

**[Table 1]**

| | Example 1 | Example 2 | Example 3 | Example 4 | Example 5 | Example 6 |
|---|---|---|---|---|---|---|
| Effectiv e ingredie nt | Donepez il | Sildena fil | Vardena fil | Sumatrip tan | dapoxet ine | Ondanset ron |
| the amount used | 10 mg | 50mg | 20mg | 50mg | 60mg | 8mg |
| bead | Sucrose bead | Sucrose bead | Sucrose bead | Sucrose bead | Sucrose bead | Sucrose bead |
| the amount used | 150 mg | 150 mg | 150 mg | 150 mg | 150 mg | 150 mg |
| coating base | HPC | HPC | HPC | HPC | HPC | HPC |
| the amount used | 10mg | 30mg | 20mg | 30mg | 30mg | 10mg |
| coating solvent | purifie d water | purifie d water | purifie d water | purified water | purifie d water | purified water |
| the amount used | 2000 mg | 2000 mg | 2000 mg | 2000 mg | 2000 mg | 2000 mg |
| post-mixing | xylitol | xylitol | xylitol | xylitol | xylitol | xylitol |
| the amount used | 330mg | 270mg | 310mg | 270mg | 260mg | 332mg |
| the total amount of solid | 500 mg | 500 mg | 500 mg | 500 mg | 500 mg | 500 mg |
| powder | | | | | | |

### Comparative examples 1 to 6

Wet granules corresponding to Examples 1 to 6 were prepared by using a High Speed Mixer. Specifically, an effective ingredient and sucrose (200 mesh) were mixed throughly and inserted into HSM, as described in Table 2, and then a binding agent dissolved in a binding liquid was inserted with stirring. The resultant obtained by high speed association for 3 minutes was sieved by 20 mesh oscillator and then dried in a 60 centigrade hot air drier. The resultant obtained was sieved by 20 mesh oscillator again and xylitol was added to it by post-mixing, mixed them for 5 minutes and then the final result was obtained.

**[Table2]**

| | Comparat ive example 1 | Comparat ive example 2 | Comparat ive example 3 | Comparat ive example 4 | Comparat ive example 5 | Comparat ive example 6 |
|---|---|---|---|---|---|---|
| Effecti ve ingredi ent | Donepezi 1 | Sildenaf il | Vardenaf il | Sumatrip tan | dapoxeti ne | Ondanset ron |
| The amount used | 10 mg | 50mg | 20mg | 50mg | 60mg | 8mg |
| excipie nt 1 | sucrose | sucrose | sucrose | sucrose | Sucrose | sucrose |
| the amount used | 150 mg | 150 mg | 150 mg | 150 mg | 150 mg | 150 mg |
| binding agent | HPC | HPC | HPC | HPC | HPC | HPC |
| the amount used | 10mg | 10mg | 10mg | 10mg | 10mg | 10mg |
| binding solutio n | purified water | purified water | purified water | purified water | purified water | purified water |
| the amount used | 200 mg | 200 mg | 200 mg | 200 mg | 200 mg | 200 mg |
| post-mixing | xylitol | xylitol | xylitol | xylitol | xylitol | xylitol |
| the amount used | 330mg | 290mg | 320mg | 290mg | 280mg | 332mg |
| the total amount of solid powder | 500 mg | 500 mg | 500 mg | 500 mg | 500 mg | 500 mg |

### Comparative examples 7 to 12

Wet granules corresponding to Examples 1 to 6 were prepared by using a High pressure Extruder.

Specifically, an effective ingredient and sucrose (200 mesh) were mixed throughly and inserted into HSM, as described in Table 3, and then a binding agent dissolved in a binding liquid was inserted with stirring. The resultant obtained by a high speed association for 3 minutes was ejected by a single dome extruder having a dome screen size of 1mm, and then dried in 60 centigrade hot air drier. The resultant obtained was sieved by the 30 mesh oscillator again and xylitol was added to it by post-mixing, mixed them for 5 minute and then the final result was obtained.

**[Table3]**

| | Comparat ive example 7 | Comparat ive example 8 | Comparat ive example 9 | Comparat ive example 10 | Comparat ive example 11 | Comparat ive example 12 |
|---|---|---|---|---|---|---|
| Effecti ve ingredi ent | Donepezi 1 | Sildenaf il | Vardenaf il | Sumatrip tan | dapoxeti ne | Ondanset ron |
| The amount used | 10 mg | 50mg | 20mg | 50mg | 60mg | 8mg |
| excipie nt 1 | sucrose | sucrose | sucrose | sucrose | sucrose | sucrose |
| The amount used | 150 mg | 150 mg | 150 mg | 150 mg | 150 mg | 150 mg |
| Binding agent | HPC | HPC | HPC | HPC | HPC | HPC |
| The amount used | 10mg | 10mg | 10mg | 10mg | 10mg | 10mg |
| Binding solutio n | purified water | purified water | purified water | purified water | purified water | purified water |
| The amount used | 200 mg | 200 mg | 200 mg | 200 mg | 200 mg | 200 mg |
| Post-mixing | xylitol | xylitol | xylitol | xylitol | xylitol | xylitol |
| The amount used | 330mg | 290mg | 320mg | 290mg | 280mg | 332mg |
| the total amount of solid powder | 500 mg | 500 mg | 500 mg | 500 mg | 500 mg | 500 mg |

### Comparative examples 13 to 18

Wet granules corresponding to Examples 1 to 6 were prepared by using a Fluid Bed Granulator. Specifically, the effective ingredient and sucrose (200 mesh) were flow-mixed in FBG. The resultant obtained by dissolving the coating base in the coating solution and then assembling the coating solution by in a spray manner, with flowing the mixture comprising the effective ingredient in FBG (the temperature of the wind: 50°C). Additionally, sugar or sugar alcohol was mixed to the resultant and then the final mixture was obtained.

**[Table 4]**

| | Comparat ive example 13 | Comparat ive example 14 | Comparat ive example 15 | Comparat ive example 16 | Comparat ive example 17 | Comparat ive example 18 |
|---|---|---|---|---|---|---|
| Effecti | Donepezi | Sildenaf | Vardenaf | Sumatrip | dapoxeti | Ondanset |
| ve ingredi ent | 1 | il | il | tan | ne | ron |
| the amount used | 10 mg | 50mg | 20mg | 50mg | 60mg | 8mg |
| excipie nt | sucrose | sucrose | sucrose | sucrose | sucrose | sucrose |
| the amount used | 150 mg | 150 mg | 150 mg | 150 mg | 150 mg | 150 mg |
| coating base | HPC | HPC | HPC | HPC | HPC | HPC |
| the amount used | 10mg | 10mg | 10mg | 10mg | 10mg | 10mg |
| coating solvent | purified water | purified water | purified water | purified water | purified water | purified water |
| the amount used | 2000 mg | 2000 mg | 2000 mg | 2000 mg | 2000 mg | 2000 mg |
| post-mixing | xylitol | xylitol | xylitol | xylitol | xylitol | xylitol |
| the amount used | 330mg | 290mg | 320mg | 290mg | 280mg | 332mg |
| the total amount of solid powder | 500 mg | 500 mg | 500 mg | 500 mg | 500 mg | 500 mg |

### Experiment example 1. Experiment for feeling of irritation in a mouth

Sensory tests by twenty (20) healthy adults were practiced for feeling of irritation in a mouth regarding the pharmaceutical compositions prepared in the above examples 1 to 6 and comparative examples 1 to 18, with melting the prepared pharmaceutical composition in the mouth. In this case, a blind-test was kept for test subjects in all tests. The results were estimated according to the below evaluation criteria and then the test results are represented.

Score 1 : No feeling of irritation.

Score 2 : Presence of feeling of irritation but little feeling of it.

Score 3 : A little feeling of irritation.

Score 4 : Presence for feeling of irritation and repulsion.

Score 5 : Very severe feeling of irritation.

The following tables 5 to 8 show the results measured by each subject.

**[Table 5]**

| Example 1 | Example 2 | Example 3 | Example 4 | Example 5 | Example 6 |
|---|---|---|---|---|---|
| 1 | 1 | 1 | 1 | 1 | 1 |
| 1 | 1 | 1 | 1 | 1 | 2 |
| 2 | 2 | 2 | 2 | 2 | 2 |
| 2 | 2 | 2 | 1 | 1 | 1 |
| 1 | 1 | 1 | 2 | 1 | 1 |
| 1 | 1 | 1 | 2 | 1 | 2 |
| 1 | 1 | 2 | 2 | 1 | 2 |
| 2 | 2 | 1 | 1 | 1 | 2 |
| 2 | 2 | 1 | 2 | 2 | 2 |
| 1 | 2 | 1 | 2 | 2 | 1 |
| 2 | 1 | 1 | 2 | 1 | 1 |
| 1 | 1 | 2 | 1 | 2 | 1 |
| 1 | 1 | 2 | 2 | 2 | 1 |
| 2 | 1 | 2 | 2 | 1 | 2 |
| 1 | 2 | 2 | 1 | 2 | 1 |
| 1 | 2 | 2 | 2 | 2 | 2 |
| 1 | 2 | 2 | 1 | 2 | 1 |
| 2 | 2 | 2 | 2 | 2 | 2 |
| 2 | 2 | 1 | 2 | 2 | 1 |
| 2 | 1 | 2 | 2 | 1 | 2 |
| 1.5 | 1.5 | 1.6 | 1.7 | 1.5 | 1.5 |

| | | | | | |
|---|---|---|---|---|---|
| (The table is listed regardless of the order of experimental subject, the number on the bottom is an arithmetic mean value) | | | | | |

**[Table 6]**

| Comparati ve example 1 | Comparati ve example 2 | Comparati ve example 3 | Comparati ve example 4 | Comparati ve example 5 | Comparati ve example 6 |
|---|---|---|---|---|---|
| 5 | 3 | 5 | 5 | 5 | 3 |
| 5 | 4 | 3 | 3 | 3 | 4 |
| 3 | 3 | 3 | 3 | 3 | 5 |
| 5 | 3 | 5 | 3 | 3 | 4 |
| 3 | 5 | 4 | 5 | 4 | 4 |
| 4 | 5 | 4 | 3 | 3 | 3 |
| 4 | 3 | 3 | 5 | 4 | 4 |
| 3 | 5 | 3 | 4 | 3 | 4 |
| 4 | 3 | 5 | 4 | 4 | 3 |
| 4 | 4 | 4 | 4 | 4 | 3 |
| 5 | 3 | 3 | 3 | 5 | 4 |
| 5 | 3 | 4 | 3 | 3 | 4 |
| 5 | 3 | 5 | 3 | 3 | 5 |
| 4 | 3 | 3 | 3 | 3 | 4 |
| 4 | 3 | 4 | 5 | 5 | 4 |
| 5 | 4 | 3 | 3 | 5 | 3 |
| 4 | 5 | 5 | 5 | 3 | 4 |
| 3 | 4 | 5 | 4 | 4 | 4 |
| 4 | 5 | 3 | 3 | 3 | 3 |
| 3 | 5 | 4 | 4 | 3 | 3 |
| 4.1 | 3.8 | 3.9 | 3.8 | 3.7 | 3.8 |

| | | | | | |
|---|---|---|---|---|---|
| (The table is listed regardless of the order of experimental subject, the number on the bottom is an arithmetic mean value) | | | | | |

**[Table 7]**

| Comparati ve example 7 | Comparati ve example 8 | Comparati ve example 9 | Comparati ve example 10 | Comparati ve example 11 | Comparati ve example 12 |
|---|---|---|---|---|---|
| 5 | 4 | 4 | 5 | 5 | 4 |
| 5 | 5 | 5 | 5 | 5 | 5 |
| 4 | 4 | 5 | 5 | 5 | 4 |
| 4 | 5 | 5 | 4 | 5 | 4 |
| 5 | 5 | 4 | 5 | 4 | 5 |
| 5 | 4 | 5 | 4 | 5 | 5 |
| 4 | 5 | 5 | 4 | 5 | 4 |
| 5 | 4 | 5 | 4 | 5 | 5 |
| 4 | 4 | 5 | 5 | 4 | 5 |
| 5 | 5 | 4 | 4 | 5 | 5 |
| 4 | 4 | 5 | 5 | 5 | 5 |
| 5 | 4 | 4 | 5 | 4 | 5 |
| 4 | 4 | 5 | 5 | 4 | 4 |
| 4 | 4 | 5 | 4 | 4 | 4 |
| 4 | 5 | 5 | 4 | 4 | 4 |
| 4 | 4 | 5 | 5 | 4 | 4 |
| 4 | 4 | 4 | 4 | 5 | 4 |
| 5 | 5 | 5 | 4 | 4 | 4 |
| 5 | 5 | 4 | 4 | 4 | 4 |
| 5 | 5 | 5 | 4 | 5 | 4 |
| 4.5 | 4.5 | 4.7 | 4.5 | 4.6 | 4.4 |

| | | | | | |
|---|---|---|---|---|---|
| (The table is listed regardless of the order of experimental subject, the number on the bottom is an arithmetic mean value) | | | | | |

**[Table 8]**

| Comparati ve example 13 | Comparati ve example 14 | Comparati ve example 15 | Comparati ve example 16 | Comparati ve example 17 | Comparati ve example 18 |
|---|---|---|---|---|---|
| 1 | 3 | 3 | 1 | 3 | 3 |
| 1 | 2 | 2 | 3 | 3 | 2 |
| 1 | 3 | 3 | 1 | 1 | 1 |
| 2 | 1 | 2 | 2 | 2 | 2 |
| 1 | 1 | 3 | 1 | 3 | 2 |
| 2 | 1 | 2 | 2 | 2 | 3 |
| 1 | 1 | 1 | 3 | 1 | 2 |
| 2 | 2 | 3 | 2 | 2 | 1 |
| 2 | 3 | 2 | 3 | 1 | 3 |
| 3 | 3 | 2 | 3 | 3 | 2 |
| 1 | 1 | 1 | 1 | 1 | 1 |
| 1 | 3 | 1 | 2 | 3 | 1 |
| 1 | 1 | 3 | 1 | 3 | 2 |
| 2 | 1 | 1 | 3 | 1 | 2 |
| 2 | 3 | 1 | 1 | 2 | 2 |
| 3 | 2 | 2 | 1 | 3 | 2 |
| 1 | 3 | 1 | 1 | 3 | 2 |
| 2 | 1 | 1 | 2 | 1 | 1 |
| 3 | 1 | 2 | 2 | 3 | 2 |
| 1 | 3 | 1 | 3 | 3 | 3 |
| 1.7 | 2.0 | 1.9 | 1.9 | 2.2 | 2.0 |

| | | | | | |
|---|---|---|---|---|---|
| (The table is listed regardless of the order of experimental subjects, the number on the bottom is an arithmetic mean value) | | | | | |

Based on the above Tables 5 to 8, after determining a significance via t-assay of Examples and Comparative examples for the same main component, the following Tables 9 to 11 were obtained.

**[Table 9]**

| Example 1 vs Comparat ive example 1 | Example 2 vs Comparat ive example 2 | Example 3 vs Comparat ive example 3 | Example 4 vs Comparati ve example 4 | Example 5 vs Comparati ve example 5 | Example 6 vs Comparat ive example 6 |
|---|---|---|---|---|---|
| p< 0.001 | p< 0.001 | p< 0.001 | p< 0.001 | p< 0.001 | p< 0.001 |

As can be seen from Table 9, it was verified that all Example 1-6 differed with a statistical significance in 95% confidence limit to Comparative examples 1~6.

**[Table 10]**

| Example 1 vs Comparati ve example 7 | Example 2 vs Comparat ive example 8 | Example 3 vs Comparat ive example 9 | Example 4 vs Comparat ive example 10 | Example 5 vs Comparati ve example 11 | Example 6 vs Comparati ve example 12 |
|---|---|---|---|---|---|
| p< 0.001 | p< 0.001 | p< 0.001 | p< 0.001 | p< 0.001 | p< 0.001 |

As can be seen from Table 10, it was verified that all Example 1-6 differed with statistical significance in 95% confidence limit to Comparative examples 1~6.

**[Table 11]**

| Example 1 vs Comparati ve example 13 | Example 2 vs Comparati ve example 14 | Example 3 vs Comparati ve example 15 | Example 4 vs Comparati ve example 16 | Example 5 vs Comparati ve example 17 | Example 6 vs Comparati ve example 18 |
|---|---|---|---|---|---|
| p=0.3283 | p=0.0689 | p=0.1702 | p=0.2624 | p=0.0043 | p=0.0241 |

Example 1 and Comparative example 13, Example 2 and Comparative example 14, Example 3 and Comparative example 15, and Example 4 and Comparative example 16 show no significant difference statistically in 95% confidence limit, but Example 5 and Comparative example 17, and Example 6 and Comparative example 18 show a significant difference statistically in 95% confidence limit.

### Experiment example 2 : Experiment for aftersensation in a mouth

Sensory tests by twenty (20) healthy adults were practiced for aftersensation in a mouth regarding the pharmaceutical compositions prepared in the above examples 1 to 6 and comparative examples 1 to 18 while melting them in the mouth. In this case, a blind-test was kept for the test subjects in all tests. The results were estimated according to the below evaluation criteria and then the test results were represented.

The following Tables 12 to 15 represent the result scored by each subject.

**[Table 12]**

| Example 1 | Example 2 | Example 3 | Example 4 | Example 5 | Example 6 |
|---|---|---|---|---|---|
| 2 | 2 | 2 | 2 | 1 | 2 |
| 2 | 2 | 2 | 2 | 1 | 1 |
| 2 | 2 | 1 | 1 | 2 | 2 |
| 1 | 2 | 1 | 2 | 1 | 1 |
| 2 | 2 | 1 | 2 | 1 | 1 |
| 2 | 2 | 2 | 1 | 2 | 2 |
| 2 | 1 | 1 | 1 | 1 | 2 |
| 1 | 2 | 1 | 2 | 2 | 2 |
| 2 | 1 | 2 | 1 | 1 | 2 |
| 2 | 1 | 2 | 2 | 1 | 2 |
| 1 | 1 | 1 | 2 | 1 | 1 |
| 2 | 2 | 2 | 1 | 1 | 1 |
| 2 | 1 | 1 | 2 | 2 | 1 |
| 2 | 2 | 1 | 2 | 1 | 2 |
| 1 | 1 | 1 | 2 | 2 | 1 |
| 1 | 1 | 2 | 2 | 1 | 2 |
| 2 | 1 | 1 | 1 | 1 | 2 |
| 1 | 1 | 2 | 1 | 1 | 1 |
| 2 | 1 | 2 | 1 | 1 | 2 |
| 1 | 1 | 2 | 2 | 2 | 1 |
| 1.7 | 1.5 | 1.5 | 1.6 | 1.3 | 1.6 |

| | | | | | |
|---|---|---|---|---|---|
| (The table is listed regardless of the order of experimental subjects, the number on the bottom is an arithmetic mean value) | | | | | |

**[Table 13]**

| Comparati ve example 1 | Comparati ve example 2 | Comparati ve example 3 | Comparati ve example 4 | Comparati ve example 5 | Comparati ve example 6 |
|---|---|---|---|---|---|
| 5 | 5 | 5 | 5 | 3 | 4 |
| 3 | 3 | 3 | 3 | 4 | 5 |
| 3 | 4 | 3 | 3 | 4 | 5 |
| 5 | 3 | 4 | 4 | 5 | 5 |
| 4 | 4 | 3 | 3 | 3 | 5 |
| 4 | 4 | 5 | 5 | 5 | 5 |
| 4 | 3 | 5 | 5 | 4 | 5 |
| 5 | 4 | 3 | 3 | 3 | 4 |
| 3 | 5 | 3 | 4 | 3 | 5 |
| 5 | 5 | 3 | 4 | 4 | 3 |
| 5 | 3 | 3 | 3 | 5 | 5 |
| 5 | 4 | 5 | 3 | 5 | 5 |
| 4 | 3 | 3 | 5 | 4 | 5 |
| 3 | 4 | 3 | 4 | 5 | 5 |
| 4 | 3 | 3 | 4 | 5 | 4 |
| 4 | 4 | 5 | 4 | 3 | 5 |
| 4 | 4 | 4 | 4 | 4 | 3 |
| 4 | 3 | 4 | 3 | 3 | 4 |
| 4 | 3 | 4 | 4 | 4 | 4 |
| 5 | 5 | 3 | 4 | 3 | 5 |
| 4.2 | 3.8 | 3.7 | 3.9 | 4.0 | 4.6 |

| | | | | | |
|---|---|---|---|---|---|
| (The table is listed regardless of the order of experimental subjects, the number on the bottom is an arithmetic mean value) | | | | | |

**[Table 14]**

| Comparati ve example 7 | Comparati ve example 8 | Comparati ve example 9 | Comparati ve example 10 | Comparati ve example 11 | Comparati ve example 12 |
|---|---|---|---|---|---|
| 4 | 4 | 5 | 5 | 5 | 5 |
| 4 | 5 | 4 | 4 | 4 | 5 |
| 4 | 4 | 5 | 5 | 5 | 4 |
| 5 | 4 | 4 | 5 | 4 | 5 |
| 4 | 4 | 5 | 4 | 4 | 5 |
| 5 | 5 | 4 | 4 | 5 | 4 |
| 4 | 4 | 4 | 5 | 5 | 4 |
| 4 | 4 | 5 | 5 | 4 | 4 |
| 5 | 5 | 4 | 5 | 5 | 5 |
| 5 | 5 | 5 | 5 | 4 | 5 |
| 4 | 4 | 4 | 5 | 4 | 4 |
| 4 | 5 | 4 | 5 | 5 | 5 |
| 5 | 4 | 4 | 4 | 4 | 5 |
| 5 | 4 | 4 | 5 | 4 | 5 |
| 4 | 5 | 5 | 4 | 5 | 5 |
| 4 | 5 | 5 | 4 | 5 | 5 |
| 5 | 5 | 4 | 5 | 5 | 4 |
| 5 | 4 | 5 | 4 | 4 | 4 |
| 5 | 4 | 4 | 5 | 5 | 5 |
| 5 | 4 | 5 | 5 | 4 | 5 |
| 4.5 | 4.4 | 4.5 | 4.7 | 4.5 | 4.7 |

| | | | | | |
|---|---|---|---|---|---|
| (The table is listed regardless of the order of experimental subjects, the number on the bottom is an arithmetic mean value) | | | | | |

**[Tablet 15]**

| Comparati ve example 13 | Comparati ve example 14 | Comparati ve example 15 | Comparati ve example 16 | Comparati ve example 17 | Comparati ve example 18 |
|---|---|---|---|---|---|
| 2 | 3 | 2 | 3 | 1 | 3 |
| 3 | 1 | 1 | 3 | 3 | 3 |
| 2 | 1 | 3 | 2 | 3 | 1 |
| 3 | 3 | 1 | 1 | 2 | 1 |
| 2 | 2 | 1 | 1 | 1 | 3 |
| 3 | 2 | 3 | 3 | 3 | 3 |
| 2 | 3 | 1 | 3 | 3 | 1 |
| 1 | 3 | 1 | 1 | 3 | 3 |
| 3 | 2 | 2 | 2 | 3 | 3 |
| 1 | 2 | 2 | 2 | 2 | 2 |
| 3 | 1 | 2 | 2 | 3 | 1 |
| 1 | 2 | 2 | 2 | 2 | 2 |
| 1 | 3 | 3 | 2 | 1 | 1 |
| 1 | 1 | 2 | 2 | 1 | 3 |
| 1 | 3 | 3 | 1 | 2 | 3 |
| 2 | 1 | 3 | 1 | 3 | 1 |
| 1 | 3 | 3 | 3 | 3 | 1 |
| 3 | 2 | 3 | 3 | 2 | 1 |
| 1 | 1 | 1 | 2 | 2 | 1 |
| 1 | 1 | 3 | 1 | 2 | 2 |
| 1.9 | 2.0 | 2.1 | 2.0 | 2.3 | 2.0 |

| | | | | | |
|---|---|---|---|---|---|
| (The table is listed regardless of the order of experimental subjects, the number on the bottom is an arithmetic mean value) | | | | | |

Based on the above Tables 12 to 15, after determining a significance via t-assay of Examples and Comparative examples for the same main component, the following Tables 16 to 18 were obtained.

**[Table 16]**

| Example 1 vs Comparati ve example 1 | Example 2 vs Comparati ve example 2 | Example 3 vs Comparati ve example 3 | Example 4 vs Comparati ve example 4 | Example 5 vs Comparati ve example 5 | Example 6 vs Comparati ve example 6 |
|---|---|---|---|---|---|
| p< 0.001 | p< 0.001 | p< 0.001 | p< 0.001 | p< 0.001 | p< 0.001 |

As can be seen from Table 16, it was verified that all Examples 1-6 differed with a statistical significance in 95% confidence limit from Comparative examples 1~6.

**[Table 17]**

| Example 1 vs Comparati ve | Example 2 vs Comparati ve | Example 3 vs Comparati ve | Example 4 vs Comparati ve | Example 5 vs Comparati ve | Example 6 vs Comparati ve |
|---|---|---|---|---|---|
| example 7 | example 8 | example 9 | example 10 | example 11 | example 12 |
| p< 0.001 | p< 0.001 | p< 0.001 | p< 0.001 | p< 0.001 | p< 0.001 |

It was verified that all Examples 1-6 differed with a statistical significance in 95% confidence limit from Comparative examples 1~6.

**[Table 18]**

| Example 1 vs Comparati ve example 13 | Example 2 vs Comparati ve example 14 | Example 3 vs Comparati ve example 15 | Example 4 vs Comparati ve example 16 | Example 5 vs Comparati ve example 17 | Example 6 vs Comparati ve example 18 |
|---|---|---|---|---|---|
| p=0.3780 | p=0.0184 | p=0.0104 | p=0.0647 | P<0.001 | p=0.1039 |

Example 1 and Comparative example 13, Example 4 and Comparative example 16, and Example 6 and Comparative example 18 showed no difference having a statistical significance in 95% confidence limit, but Example 2 and Comparative example 14, Example 3 and Comparative example 15, and Example 5 and Comparative example 17 showed the difference have a statistical significance in 95% confidence limit.

### Experiment example 3 : Experiment for the dissolution rate in a mouth

Dissolution rate in a mouth by twenty (20) healthy adults was determined regarding the pharmaceutical compositions prepared in the above examples 1 to 6 and comparative examples 1 to 18 with melting them in the mouth (determination unit: second). In this case, a blind-test was kept for the test subjects in all tests.

The test results by scored by each subject were shown in below Tables 19 to 22.

**[Table 19]**

| Example 1 | Example 2 | Example 3 | Example 4 | Example 5 | Example 6 |
|---|---|---|---|---|---|
| 7 | 5 | 8 | 6 | 3 | 7 |
| 5 | 5 | 6 | 7 | 3 | 6 |
| 5 | 6 | 5 | 8 | 8 | 7 |
| 6 | 5 | 8 | 8 | 6 | 4 |
| 6 | 3 | 5 | 4 | 5 | 8 |
| 5 | 4 | 7 | 5 | 7 | 6 |
| 8 | 8 | 8 | 3 | 5 | 8 |
| 7 | 5 | 5 | 7 | 6 | 7 |
| 8 | 5 | 5 | 4 | 5 | 4 |
| 5 | 6 | 7 | 6 | 5 | 8 |
| 4 | 6 | 8 | 4 | 8 | 4 |
| 8 | 3 | 5 | 3 | 3 | 3 |
| 3 | 4 | 3 | 4 | 4 | 6 |
| 6 | 6 | 5 | 5 | 3 | 6 |
| 7 | 3 | 6 | 4 | 5 | 7 |
| 7 | 5 | 3 | 5 | 4 | 5 |
| 5 | 8 | 4 | 5 | 8 | 5 |
| 4 | 5 | 4 | 8 | 8 | 3 |
| 3 | 6 | 8 | 8 | 4 | 6 |
| 4 | 5 | 5 | 7 | 3 | 5 |
| 5.7 | 5.2 | 5.8 | 5.6 | 5.2 | 5.8 |

| | | | | | |
|---|---|---|---|---|---|
| (The table is listed regardless of the order of experimental subjects, the number on the bottom is an arithmetic mean value) | | | | | |

**[Table 20]**

| Comparati ve example 1 | Comparati ve example 2 | Comparati ve example 3 | Comparati ve example 4 | Comparati ve example 5 | Comparati ve example 6 |
|---|---|---|---|---|---|
| 27 | 30 | 27 | 17 | 15 | 23 |
| 20 | 22 | 30 | 23 | 16 | 23 |
| 15 | 23 | 21 | 23 | 21 | 25 |
| 29 | 15 | 19 | 20 | 25 | 22 |
| 24 | 22 | 16 | 29 | 28 | 25 |
| 16 | 28 | 22 | 30 | 16 | 19 |
| 18 | 22 | 27 | 18 | 21 | 18 |
| 26 | 25 | 21 | 22 | 24 | 19 |
| 19 | 18 | 18 | 28 | 23 | 18 |
| 20 | 30 | 15 | 28 | 16 | 16 |
| 22 | 19 | 30 | 30 | 23 | 28 |
| 27 | 27 | 19 | 28 | 15 | 29 |
| 17 | 15 | 15 | 26 | 27 | 29 |
| 20 | 17 | 17 | 17 | 24 | 23 |
| 23 | 23 | 26 | 19 | 30 | 23 |
| 29 | 16 | 15 | 23 | 17 | 21 |
| 28 | 24 | 21 | 24 | 20 | 29 |
| 15 | 22 | 22 | 17 | 17 | 30 |
| 29 | 27 | 26 | 26 | 27 | 21 |
| 15 | 15 | 23 | 25 | 24 | 29 |
| 22.0 | 22.0 | 21.5 | 23.7 | 21.5 | 23.5 |

**[Table 21]**

| Comparati ve example 7 | Comparati ve example 8 | Comparati ve example 9 | Comparati ve example 10 | Comparati ve example 11 | Comparati ve example 12 |
|---|---|---|---|---|---|
| 57 | 51 | 47 | 57 | 41 | 26 |
| 49 | 34 | 56 | 57 | 34 | 45 |
| 56 | 59 | 52 | 50 | 31 | 60 |
| 35 | 46 | 53 | 51 | 52 | 47 |
| 40 | 43 | 51 | 28 | 53 | 55 |
| 41 | 54 | 35 | 39 | 32 | 59 |
| 35 | 54 | 25 | 27 | 31 | 57 |
| 34 | 27 | 27 | 25 | 35 | 47 |
| 26 | 33 | 56 | 50 | 57 | 55 |
| 35 | 46 | 48 | 41 | 49 | 58 |
| 51 | 42 | 56 | 56 | 31 | 28 |
| 56 | 39 | 57 | 25 | 47 | 51 |
| 27 | 46 | 25 | 54 | 35 | 50 |
| 26 | 37 | 41 | 40 | 58 | 42 |
| 40 | 39 | 58 | 58 | 33 | 50 |
| 59 | 26 | 29 | 39 | 38 | 32 |
| 34 | 34 | 50 | 59 | 36 | 59 |
| 39 | 26 | 25 | 54 | 43 | 50 |
| 54 | 36 | 43 | 29 | 45 | 49 |
| 41 | 37 | 55 | 37 | 56 | 35 |
| 41.8 | 40.5 | 44.5 | 43.8 | 41.9 | 47.8 |

**[Table 22]**

| Comparative example | Comparative example | Comparative example | Comparative example | Comparative example | Comparative example |
|---|---|---|---|---|---|
| 13 | 14 | 15 | 16 | 17 | 18 |
| 9 | 12 | 14 | 9 | 12 | 8 |
| 9 | 8 | 10 | 10 | 13 | 9 |
| 12 | 11 | 15 | 12 | 10 | 10 |
| 14 | 10 | 14 | 8 | 12 | 9 |
| 14 | 14 | 9 | 14 | 11 | 10 |
| 12 | 11 | 14 | 15 | 8 | 11 |
| 14 | 13 | 10 | 8 | 11 | 14 |
| 14 | 15 | 11 | 13 | 12 | 9 |
| 9 | 13 | 15 | 8 | 14 | 9 |
| 12 | 15 | 9 | 11 | 10 | 15 |
| 9 | 8 | 8 | 9 | 14 | 12 |
| 10 | 11 | 13 | 11 | 8 | 8 |
| 9 | 10 | 9 | 13 | 12 | 14 |
| 14 | 14 | 10 | 10 | 8 | 8 |
| 15 | 15 | 11 | 10 | 8 | 8 |
| 9 | 14 | 12 | 15 | 8 | 11 |
| 14 | 10 | 15 | 13 | 15 | 10 |
| 10 | 11 | 15 | 10 | 9 | 11 |
| 8 | 8 | 15 | 9 | 15 | 10 |
| 13 | 13 | 15 | 8 | 13 | 13 |
| 11.5 | 11.8 | 12.2 | 10.8 | 11.2 | 10.5 |

| | | | | | |
|---|---|---|---|---|---|
| (The table is listed regardless of the order of experimental subjects, the number on the bottom is an arithmetic mean value) | | | | | |

Based on tablets 19 to 22 as above, when determining the significance for the same component through t-assay for examples and comparative examples, the below Tables 23 to 25 were obtained.

**[Table 23]**

| Example 1 vs Comparati ve example 1 | Example 2 vs Comparati ve example 2 | Example 3 vs Comparati ve example 3 | Example 4 vs Comparati ve example 4 | Example 5 vs Comparati ve example 5 | Example 6 vs Comparati ve example 6 |
|---|---|---|---|---|---|
| p< 0.001 | p< 0.001 | p< 0.001 | p< 0.001 | p< 0.001 | p< 0.001 |

It was verified that all Examples 1-6 differed with a statistical significance in 95% confidence limit from Comparative examples 1~6.

**[Table 24]**

| Example1 vs Comparati ve example 7 | Example 2 vs Comparati ve example 8 | Example 3 vs Comparati ve example 9 | Example 4 vs Comparati ve example 10 | Example 5 vs Comparati ve example 11 | Example 6 vs Comparati ve example 12 |
|---|---|---|---|---|---|
| p< 0.001 | p< 0.001 | p< 0.001 | p< 0.001 | p< 0.001 | p< 0.001 |

As can be seen from Table 24, it was verified that all Examples 1-6 differed with a statistical significance in 95% confidence limit from Comparative examples 7~12.

**[Table 25]**

| Example 1 vs Comparati ve example | Example 2 vs Comparati ve example | Example 3 vs Comparat ive example | Example 4 vs Comparat ive example | Example 5 vs Comparati ve example | Example 6 vs Comparati ve example |
|---|---|---|---|---|---|
| 13 | 14 | 15 | 16 | 17 | 18 |
| p< 0.001 | p< 0.001 | p< 0.001 | p< 0.001 | p< 0.001 | p< 0.001 |

As can be seen from Table 25, it was verified that all Examples 1-6 differed with a statistical significance in 95% confidence limit from Comparative examples 13~18.

### Experiment example 4 : Evaluation for the amount remained in the wrapping paper

Pharmaceutical compositions prepared in Examples 1 to 6 and Comparative examples 1 to 18 were packaged into each of 100 raws by using 6 rows sachet wrapping machine having the size of 5cm x 7cm. Among these, 10 specimens of the third raw positioned on the very middle were taken and the drug in the wrapping paper was taken out at once. After measuring the weight for the wrapping paper with fine powders, all content remained in the wrapping paper were removed and then again the weight for wrapping paper without fine powders was determined and it was represented as the ratio of all contents. (unit: %)

The below tables 26 to 29 show the test result for each specimen.

**[Table 26]**

| Example 1 | Example 2 | Example 3 | Example 4 | Example 5 | Example 6 |
|---|---|---|---|---|---|
| 0.4% | 0.4% | 0.6% | 0.8% | 0.9% | 0.6% |
| 0.8% | 0.8% | 0.4% | 0.8% | 0.9% | 0.8% |
| 0.3% | 0.3% | 0.5% | 0.3% | 0.3% | 0.4% |
| 0.6% | 0.5% | 0.5% | 0.4% | 1.0% | 0.4% |
| 0.5% | 0.6% | 0.6% | 0.8% | 0.9% | 0.6% |
| 0.3% | 0.4% | 0.3% | 0.9% | 0.6% | 0.8% |
| 0.8% | 0.4% | 1.0% | 0.4% | 0.4% | 0.5% |
| 0.9% | 0.4% | 0.7% | 0.6% | 0.3% | 0.4% |
| 0.8% | 0.5% | 0.4% | 0.5% | 0.5% | 0.6% |
| 0.5% | 0.9% | 0.6% | 0.4% | 0.3% | 0.7% |
| 0.6% | 0.5% | 0.6% | 0.6% | 0.6% | 0.6% |

| | | | | | |
|---|---|---|---|---|---|
| (The number on the bottom represents an arithmetic mean value) | | | | | |

**[Table 27]**

| Comparati ve example 1 | Comparati ve example 2 | Comparati ve example 3 | Comparati ve example 4 | Comparati ve example 5 | Comparati ve example 6 |
|---|---|---|---|---|---|
| 1.5% | 1.9% | 1.7% | 1.0% | 1.5% | 1.1% |
| 1.1% | 1.5% | 1.7% | 1.2% | 1.5% | 1.4% |
| 1.6% | 2.0% | 2.4% | 1.8% | 2.5% | 1.3% |
| 2.0% | 1.4% | 2.0% | 2.0% | 1.2% | 1.3% |
| 2.4% | 1.7% | 1.9% | 1.0% | 1.3% | 1.5% |
| 1.2% | 1.9% | 1.3% | 2.4% | 1.8% | 2.0% |
| 2.3% | 2.5% | 2.2% | 1.7% | 1.1% | 1.4% |
| 1.9% | 2.2% | 2.4% | 1.5% | 2.3% | 2.5% |
| 2.3% | 2.1% | 1.4% | 2.3% | 1.7% | 1.3% |
| 1.3% | 1.9% | 1.8% | 1.0% | 1.5% | 1.3% |
| 1.8% | 1.9% | 1.9% | 1.6% | 1.6% | 1.5% |

| | | | | | |
|---|---|---|---|---|---|
| (The number on the bottom represents an arithmetic mean value) | | | | | |

**[Table 28]**

| Comparati ve example 7 | Comparati ve example 8 | Comparati ve example 9 | Comparati ve example 10 | Comparati ve example 11 | Comparati ve example 12 |
|---|---|---|---|---|---|
| 1.4% | 1.0% | 0.8% | 1.8% | 1.4% | 2.0% |
| 1.2% | 0.9% | 1.4% | 1.6% | 0.8% | 2.0% |
| 1.3% | 1.5% | 1.8% | 0.8% | 1.3% | 1.2% |
| 1.9% | 1.3% | 0.9% | 1.1% | 1.7% | 1.6% |
| 1.8% | 1.4% | 1.6% | 1.6% | 1.6% | 1.1% |
| 1.0% | 0.9% | 1.7% | 1.6% | 1.1% | 1.0% |
| 0.9% | 0.8% | 1.5% | 1.0% | 0.9% | 1.6% |
| 1.3% | 1.9% | 1.3% | 1.2% | 0.8% | 1.6% |
| 0.8% | 1.7% | 1.0% | 1.8% | 2.0% | 1.2% |
| 1.4% | 1.6% | 1.7% | 1.0% | 1.2% | 1.9% |
| 1.3% | 1.3% | 1.4% | 1.4% | 1.3% | 1.5% |

| | | | | | |
|---|---|---|---|---|---|
| (The number on the bottom represents an arithmetic mean value) | | | | | |

**[Table 29]**

| Comparati ve example 13 | Comparati ve example 14 | Comparati ve example 15 | Comparati ve example 16 | Comparati ve example 17 | Comparati ve example 18 |
|---|---|---|---|---|---|
| 3.6% | 2.8% | 1.8% | 3.8% | 2.1% | 2.7% |
| 2.8% | 3.1% | 2.6% | 3.4% | 2.8% | 2.5% |
| 3.2% | 3.3% | 1.6% | 2.5% | 2.7% | 3.8% |
| 3.1% | 3.4% | 3.6% | 2.3% | 2.5% | 2.9% |
| 2.9% | 1.8% | 3.3% | 4.0% | 2.3% | 1.7% |
| 2.8% | 2.9% | 3.6% | 2.3% | 2.2% | 3.9% |
| 1.6% | 3.4% | 1.8% | 3.8% | 1.8% | 2.3% |
| 3.4% | 2.0% | 3.5% | 2.2% | 1.7% | 4.0% |
| 1.6% | 1.6% | 3.9% | 3.3% | 3.7% | 1.9% |
| 2.5% | 3.1% | 3.6% | 1.7% | 2.3% | 3.9% |
| 2.7% | 2.8% | 2.9% | 2.9% | 2.4% | 3.0% |

| | | | | | |
|---|---|---|---|---|---|
| (The number on the bottom represents an arithmetic mean value) | | | | | |

Based on the above Tables 26 to 29, when determining the significance for the same main component via t-assay for examples and comparative examples, the below Tables 30 to 32 were obtained.

**[Table 30]**

| Example 1 vs Comparati ve example 1 | Example 2 vs Comparati ve example 2 | Example 3 vs Comparati ve example 3 | Example 4 vs Comparati ve example 4 | Example5 vs Comparati ve example 5 | Example 6 vs Comparati ve example 6 |
|---|---|---|---|---|---|
| P< 0.001 | p< 0.001 | p< 0.001 | p< 0.001 | p< 0.001 | p< 0.001 |

As can be seen from Table 30, it was verified that all Examples 1~6 differed with a statistical significance in 95% confidence limit from Comparative examples 1~6.

**[Table 31]**

| Example 1 vs Comparati ve example 7 | Example 2 vs Comparati ve example 8 | Example 3 vs Comparati ve example 9 | Example 4 vs Comparati ve example 10 | Example 5 vs Comparati ve example 11 | Example 6 vs Comparati ve example 12 |
|---|---|---|---|---|---|
| P< 0.001 | p< 0.001 | p< 0.001 | p< 0.001 | p< 0.001 | p< 0.001 |

As can be seen from Table 31, it was verified that all Examples 1~6 differed with a statistical significance in 95% confidence limit from Comparative examples 7~12.

**[Table 32]**

| Example 1 vs Comparati ve example 13 | Example 2 vs Comparati ve example 14 | Example 3 vs Comparati ve example 15 | Example 4 vs Comparati ve example 16 | Example 5 vs Comparati ve example 17 | Example 6 vs Comparati ve example 18 |
|---|---|---|---|---|---|
| P< 0.001 | p< 0.001 | p< 0.001 | p< 0.001 | p< 0.001 | p< 0.001 |

As can be seen from Table 32, it was verified that all Examples 1~6 differed with a statistical significance in 95% confidence limit from Comparative examples 13~18.

### Experiment example 5. Estimation for the uniformity of contents

The pharmaceutical compositions prepared by the above Examples 1 to 6 and Comparative examples 1 to 18 were packaged into each of 100 raws by using 6 rows sachet wrapping machine having the size of 5cm x 7cm. Among these, 10 specimens were randomly taken and the amounts of the contents were measured according to the test method for the uniformity of contents in the preparation uniformity test of the pharmacopoeia general test method. Standard deviations for contents of the specimen were calculated and the determination value for the uniformity of contents was calculated by multiplying a decision coefficient (k) 2.4 when specimens were 10. The below Tables 33 to 35 show the result of measurement of each specimen.

**[Table 33]**

| | Example 1 | Example 2 | Example 3 | Example 4 | Example 5 | Example 6 |
|---|---|---|---|---|---|---|
| specimen1 | 101.1% | 99.1% | 98.7% | 99.8% | 100.6% | 99.0% |
| specimen2 | 100.1% | 101.4% | 98.3% | 101.7% | 98.8% | 98.7% |
| Specimen 3 | 97.2% | 97.8% | 101.3% | 99.6% | 101.5% | 98.6% |
| Specimen 4 | 100.5% | 101.2% | 101.2% | 100.5% | 98.5% | 99.7% |
| Specimen 5 | 98.9% | 97.6% | 98.0% | 98.9% | 99.0% | 99.7% |
| Specimen 6 | 97.4% | 99.7% | 100.4% | 100.6% | 100.9% | 101.9% |
| Specimen 7 | 100.8% | 100.4% | 100.5% | 98.5% | 100.9% | 100.0% |
| Specimen 8 | 100.5% | 99.3% | 101.1% | 99.4% | 97.8% | 100.4% |
| Specimen 9 | 97.9% | 97.1% | 97.5% | 97.5% | 100.1% | 101.7% |
| Specimen 10 | 100.7% | 98.2% | 98.0% | 99.0% | 99.2% | 99.2% |
| Average | 99.5% | 99.2% | 99.5% | 99.6% | 99.7% | 99.9% |
| Standard deviation | 1.4% | 1.4% | 1.5% | 1.1% | 1.2% | 1.1% |
| Determination value | 3.4% | 3.4% | 3.5% | 2.7% | 2.8% | 2.6% |

**[Table 34]**

| | Compara tive example 1 | Compara tive example 2 | Compara tive example 3 | Compara tive example 4 | Compara tive example 5 | Compara tive example 6 |
|---|---|---|---|---|---|---|
| Specimen 1 | 101.8% | 106.7% | 95.5% | 107.0% | 92.6% | 93.3% |
| Specimen 2 | 99.1% | 97.0% | 95.5% | 107.4% | 96.1% | 105.3% |
| Specimen 3 | 98.9% | 105.7% | 103.8% | 93.2% | 98.6% | 103.0% |
| Specimen 4 | 104.9% | 93.6% | 104.1% | 106.6% | 100.7% | 101.4% |
| Specimen 5 | 93.0% | 101.9% | 92.2% | 99.5% | 107.9% | 99.8% |
| Specimen 6 | 98.0% | 105.9% | 97.1% | 104.8% | 107.8% | 93.1% |
| Specimen 7 | 93.0% | 106.3% | 92.8% | 107.4% | 95.8% | 93.3% |
| Specimen 8 | 93.3% | 98.6% | 106.3% | 101.4% | 107.4% | 93.0% |
| Specimen 9 | 104.9% | 101.5% | 103.4% | 107.6% | 97.3% | 94.8% |
| Specimen 10 | 102.4% | 97.6% | 100.3% | 92.4% | 101.5% | 108.0% |
| Average | 98.9% | 101.5% | 99.1% | 102.7% | 100.6% | 98.5% |
| Standard deviation | 4.4% | 4.4% | 4.9% | 5.6% | 5.2% | 5.4% |
| Determinatio nvalue | 10.6% | 10.6% | 11.6% | 13.5% | 12.6% | 13.0% |

**[Table 35]**

| | Compara tive example 7 | Compara tive example 8 | Compara tive example 9 | Compara tive example 10 | Compara tive example 11 | Compara tive example 12 |
|---|---|---|---|---|---|---|
| Specimen 1 | 104.4% | 102.6% | 107.0% | 102.0% | 99.7% | 101.0% |
| Specimen 2 | 105.9% | 98.4% | 103.4% | 101.6% | 95.9% | 101.2% |
| Specimen 3 | 96.7% | 94.0% | 100.6% | 103.1% | 99.7% | 93.1% |
| Specimen 4 | 96.9% | 94.5% | 98.3% | 106.8% | 106.5% | 103.5% |
| Specimen 5 | 106.7% | 99.5% | 102.7% | 94.2% | 93.3% | 96.5% |
| Specimen 6 | 93.9% | 97.5% | 97.5% | 97.5% | 96.4% | 99.2% |
| Specimen 7 | 98.6% | 99.0% | 102.4% | 100.2% | 105.4% | 100.0% |
| Specimen 8 | 94.4% | 104.2% | 103.5% | 97.8% | 96.7% | 94.3% |
| Specimen 9 | 95.8% | 93.4% | 97.0% | 98.0% | 93.9% | 97.8% |
| Specimen 10 | 105.7% | 97.5% | 105.7% | 96.8% | 99.0% | 99.4% |
| Average | 99.9% | 98.0% | 101.8% | 99.8% | 98.6% | 98.6% |
| Standard deviation | 4.9% | 3.4% | 3.2% | 3.5% | 4.2% | 3.1% |
| Determina tion value | 11.8% | 8.1% | 7.7% | 8.4% | 10.1% | 7.4% |

**[Table 36]**

| | Compara tive example 13 | Compara tive example 14 | Compara tive example 15 | Compara tive example 16 | Compara tive example 17 | Compara tive example 18 |
|---|---|---|---|---|---|---|
| Specimen 1 | 98.7% | 108.9% | 95.4% | 94.0% | 99.7% | 96.1% |
| Specimen 2 | 100.2% | 94.5% | 103.7% | 108.5% | 105.9% | 99.4% |
| Specimen 3 | 106.1% | 103.2% | 99.9% | 95.8% | 101.6% | 105.1% |
| Specimen 4 | 105.3% | 96.0% | 102.1% | 93.1% | 106.1% | 109.8% |
| Specimen 5 | 109.5% | 99.1% | 102.2% | 100.7% | 108.9% | 101.6% |
| Specimen 6 | 97.0% | 106.8% | 104.4% | 91.3% | 103.4% | 93.2% |
| Specimen 7 | 96.4% | 98.8% | 96.1% | 94.7% | 101.7% | 91.6% |
| Specimen 8 | 106.0% | 101.5% | 109.4% | 90.8% | 108.5% | 95.9% |
| Specimen 9 | 107.5% | 102.3% | 106.5% | 94.2% | 102.0% | 95.0% |
| Specimen 10 | 103.7% | 91.5% | 105.3% | 109.9% | 99.7% | 108.2% |
| Average | 103.1% | 100.3% | 102.5% | 97.3% | 103.8% | 99.6% |
| Standard deviation | 4.4% | 5.1% | 4.2% | 6.5% | 3.2% | 6.0% |
| Determina tion value | 10.5% | 12.3% | 10.0% | 15.5% | 7.8% | 14.5% |

As can be seen from Tables 33 to 36, Examples 1~6 show the determination value below 5 and have very excellent content uniformity, but Comparative examples 1 to 17 show high values between 7.4 to 15.5 and have the content uniformity which is not good.

In order to evaluate above example results overall, the below Tables 38 to 41 represent the overall evaluation according to the estimation criterion represented on the below Table 37.

**[Table 37]**

| | Feeling of irritation | After sensation | Dissolution rate | Occurrence of | content uniformity |
|---|---|---|---|---|---|
| | | | | remaining material | |
| ⊚ | 1≤ V <2 | 1≤ V <2 | 0 second≤ V <10 seconds | 0%≤ V <1% | 0≤ V <5 |
| ○ | 2≤ V <3 | 2≤ V <3 | 10 seconds≤ V <20 seconds | 1%≤ V <2% | 5≤ V <10 |
| Δ | 3≤ V <4 | 3≤ V <4 | 20seconds≤ V <30 seconds | 2%≤ V <3% | 10≤ V <15 |
| X | 4≤ V ≤5 | 4≤ V ≤5 | 30 seconds≤ V | 3%≤ V | 15≤ V |

| | | | | | |
|---|---|---|---|---|---|
| (V : result value estimated in each items) | | | | | |

**[Table 38]**

| Example Group | Feeling of irritatio n | After sensatio n | Dissolutio n rate | Occurrenc e of remaining material | content uniformit y |
|---|---|---|---|---|---|
| Example 1 | ⊚ | ⊚ | ⊚ | ⊚ | ⊚ |
| Example 2 | ⊚ | ⊚ | ⊚ | ⊚ | ⊚ |
| Example 3 | ⊚ | ⊚ | ⊚ | ⊚ | ⊚ |
| Example 4 | ⊚ | ⊚ | ⊚ | ⊚ | ⊚ |
| Example 5 | ⊚ | ⊚ | ⊚ | ⊚ | ⊚ |
| Example 6 | ⊚ | ⊚ | ⊚ | ⊚ | ⊚ |

**[Table 39]**

| Comparati ve example | Feeling of irritati on | Aftersensati on | Dissoluti on rate | Occurren ce of remainin g material | content uniformi ty |
|---|---|---|---|---|---|
| Comparati ve example 1 | X | X | Δ | ○ | Δ |
| Comparati ve example 2 | Δ | Δ | Δ | ○ | Δ |
| Comparati ve example 3 | Δ | Δ | Δ | ○ | Δ |
| Comparati ve example 4 | Δ | Δ | Δ | ○ | Δ |
| Comparati ve example 5 | Δ | X | Δ | ○ | Δ |
| Comparati ve example 6 | Δ | X | Δ | ○ | Δ |

**[Table 40]**

| Comparati ve example | Feeling of irritat in | Aftersensati on | Dissoluti on rate | Occurren ce of remainin g material | content uniformi ty |
|---|---|---|---|---|---|
| Comparati ve example 7 | X | X | X | ○ | Δ |
| Comparati ve example 8 | X | X | X | ○ | ○ |
| Comparati ve example 9 | X | X | X | ○ | ○ |
| Comparati ve example 10 | X | X | X | ○ | ○ |
| Comparati ve example 11 | X | X | X | ○ | Δ |
| Comparati ve example 12 | X | X | X | ○ | ○ |

**[Table 41]**

| Comparati ve example | Feeling of irritati on | Aftersensati on | Dissoluti on rate | Occurren ce of remainin g material | content uniformi ty |
|---|---|---|---|---|---|
| Comparati ve example 13 | ⊚ | ⊚ | ○ | Δ | Δ |
| Comparati ve example 14 | ○ | ○ | ○ | Δ | Δ |
| Comparati ve example 15 | ⊚ | ○ | ○ | Δ | Δ |
| Comparati ve example 16 | ⊚ | ○ | ○ | Δ | X |
| Comparati ve example 17 | ○ | ○ | ○ | Δ | ○ |
| Comparati ve example 18 | ○ | ○ | ○ | X | Δ |

### Industrial Availability

A microgranule preparation of the present invention minimizes the amounts of the preparation remained in the wrapping paper and at the same time it secures the content uniformity of the preparation included in each unit of the wrapping paper.

In addition, the microgranule preparation which shows the rapid dissolution rate in a mouth and has no aftersensation and feeling of irritation in the mouth is provided. In particular, since the preparation of the present invention can achieve the effect of the invention regardless of the kinds of the effective ingredients, it can be applied to various effective ingredients. In addition, since the manufacturing process for it is relatively simple, a process with a high efficiency can be achieved by using low cost.

## Claims

1. A composition of microgranule preparation for oral administration, consisting of
(i) a microgranule consisting of an inert core and a drug layer, wherein the inert core is coated with the drug layer, and wherein the drug layer consists of an effective ingredient and coating base; and
(ii) sugar or sugar alcohol, which is mixed with the microgranule,
wherein the effective ingredient is selected from the group consisting of Sildenafil, Tadalafil, Udenafil, Donepezil, Glymepide, Dexibuprofen, Pranlukast, Desmopressin, Acetaminophen, Pitavastatin, Rebamipide, Azithromycin, Pseudoephedrine HCl, Ranitidine HCl, and Levocetirizine HCl.

2. The composition of microgranule preparation according to claim 1, wherein the inert core is sugar or sugar alcohol.

3. The composition of microgranule preparation according to claim 1 or 2,
wherein the sugar or sugar alcohol is selected from the group consisting of xylitol, mannitol, isomalt, sorbitol, maltitol, the refined white sucrose, lactose, inositol, erythritol, crystalline fructose, trehalose, ribitol, arabitol, galactitol, lactitol and maltotritol.

4. The composition of microgranule preparation according to any one of claims 1 to 3 wherein said microgranule preparation rapidly dissolves when administered orally without water.

## Patentansprüche

1. Zusammensetzung einer mikrogranulären Zubereitung für die orale Verabreichung, bestehend aus
(i) einer Mikrogranalie, bestehend aus einem inerten Kern und einer Arzneistoffschicht, wobei der inerte Kern mit der Arzneistoffschicht überzogen ist und wobei die Arzneistoffschicht aus einem Wirkstoff und einer Überzugsgrundlage besteht; und
(ii) einem Zucker oder Zuckeralkohol, der mit der Mikrogranalie vermischt ist,
wobei der Wirkstoff ausgewählt ist aus der Gruppe, die besteht aus Sildenafil, Tadalafil, Udenafil, Donepezil, Glymepid, Dexibuprofen, Pranlukast, Desmopressin, Acetaminophen, Pitavastatin, Rebamipid, Azithromycin, Pseudoephedrin-HCl, Ranitidin-HCl und Levocetirizin-HCL.

2. Zusammensetzung einer mikrogranulären Zubereitung nach Anspruch 1, wobei es sich beim inerten Kern um einen Zucker oder Zuckeralkohol handelt.

3. Zusammensetzung einer mikrogranulären Zubereitung nach Anspruch1 oder 2, wobei der
Zucker oder Zuckeralkohol ausgewählt ist aus der Gruppe, die besteht aus Xylit, Mannit, Isomalt, Sorbit, Maltit, raffinierter weißer Saccharose, Lactose, Inosit, Erythrit, kristalliner Fructose, Trehalose, Ribit, Arabit, Galactit, Lactit und Maltotritol.

4. Zusammensetzung einer mikrogranulären Zubereitung nach einem der Ansprüche 1 bis 3, wobei sich die mikrogranuläre Zubereitung rasch löst, wenn sie auf oralem Wege ohne Wasser verabreicht wird.

## Revendications

1. Une composition de préparation de microgranule pour une administration par voie orale constituée de
(i) un microgranule constitué d'un noyau inerte et d'une couche de médicament, le noyau inerte étant revêtu avec la couche de médicament et la couche de médicament étant constituée d'un ingrédient efficace et d'une base de revêtement ; et
(ii) un sucre ou un alcool de sucre, qui est mélangé avec le microgranule,
dans laquelle l'ingrédient efficace est choisi dans le groupe constitué par le sildénafil, le tadalafil, l'udénafil, le donépézil, le glymépide, le dexibuprofène, le pranlukast, la desmopressine, l'acétaminophène, la pitavastatine, le rébamipide, l'azithromycine, l'HCI de pseudoéphédrine, l'HCl de ranitidine et l'HCl de lévocétirizine.

2. La composition de préparation de microgranule selon la revendication 1, dans laquelle le noyau inerte est un sucre ou un alcool de sucre.

3. La composition de préparation de microgranule selon la revendication 1 ou 2, dans laquelle le sucre ou l'alcool de sucre est choisi dans le groupe constitué par le xylitol, le mannitol, l'isomalt, le sorbitol, le maltitol, le saccharose blanc raffiné, le lactose, l'inositol, l'érythritol, le fructose cristallin, le tréhalose, le ribitol, l'arabitol, le galactitol, le lactitol et le maltotritol.

4. La composition de préparation de microgranule selon l'une quelconque des revendications 1 à 3 dans laquelle ladite préparation de microgranule se dissous rapidement lorsqu'elle est administrée par voie orale sans eau.
